(19) Europäisches Patentamt
European Patent Office
Office européen des brevets

(11) **EP 2 092 885 B1**

(12) **FASCICULE DE BREVET EUROPEEN**

(45) Date de publication et mention de la délivrance du brevet:
**28.01.2015 Bulletin 2015/05**

(51) Int Cl.:
*A61B 5/11* (2006.01)    *G06K 9/00* (2006.01)
*A61B 5/0205* (2006.01)    *A61N 1/37* (2006.01)
*A61N 1/365* (2006.01)

(21) Numéro de dépôt: **09290116.4**

(22) Date de dépôt: **18.02.2009**

(54) **Dispositif d'analyse d'un signal d'accélération endocardiaque**

Analysevorrichtung eines endokardialen Beschleunigungssignals

Device for analysing an endocardiac acceleration signal

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **20.02.2008 FR 0800907**

(43) Date de publication de la demande:
**26.08.2009 Bulletin 2009/35**

(73) Titulaire: **Ela Medical**
**92541 Montrouge (FR)**

(72) Inventeurs:
• **Giorgis, Lionel**
**35000 Rennes (FR)**
• **Hernandez, Alfredo**
**35700 Rennes (FR)**

(74) Mandataire: **Dupuis-Latour, Dominique et al**
**Bardehle Pagenberg**
**10, boulevard Haussmann**
**75009 Paris (FR)**

(56) Documents cités:
EP-A- 1 741 387    US-A1- 2003 060 723
US-A1- 2006 020 294    US-A1- 2007 239 218
US-B1- 7 139 609

• **BOMBARDINI T ET AL: "PEAK ENDOCARDIAL ACCELERATION REFLECTS HEART CONTRACTILITY ALSO IN ATRIAL FIBRILLATION" PACE - PACING AND CLINICAL ELECTROPHYSIOLOGY, BLACWELL FUTURA PUBLISHING, MALDEN, MA, US, vol. 23, no. 9, 1 septembre 2000 (2000-09-01), pages 1381-1385, XP000975589 ISSN: 0147-8389**
• **PLICCHI G ET AL: "PEA I and PEA II based implantable haemodynamic monitor: pre clinical studies in sheep." EUROPACE : JOURNAL OF THE EUROPEAN SOCIETY OF CARDIOLOGY, vol. 4, no. 1, 2002, pages 49-54, XP002498482 ISSN: 1099-5129**

**Description**

L'invention concerne le traitement et l'analyse des signaux d'accélération endocardiaque.

Elle est utilisable notamment dans le cadre de l'évaluation de l'efficacité, et de la recherche de configuration optimale, d'un dispositif mettant en oeuvre une technique dite CRT (*Cardiac Resynchronization Therapy*) ou BVP (Bi-Ventricular Pacing). Cette application n'est cependant pas limitative de l'invention, qui peut être utilisée à d'autres fins, de diagnostic et/ou de pilotage de dispositifs médicaux actifs.

Dans le cas particulier des dispositifs CRT, on implante au patient un appareil muni d'électrodes permettant de stimuler l'un et l'autre ventricules. Le dispositif est capable de surveiller en continu le rythme cardiaque et délivrer si nécessaire au coeur des impulsions électriques pour stimuler conjointement les ventricules gauche et droit afin de resynchroniser ces derniers. À cet effet, le dispositif applique entre les deux stimulations ventriculaires un délai interventriculaire variable, positif ou négatif, ajusté de manière à resynchroniser la contraction des ventricules avec optimisation fine de l'état hémodynamique du patient.

Un tel stimulateur CRT est par exemple divulgué dans le EP 1 108 446 A1 (ELA Medical).

Il est essentiel, dans la mise en oeuvre d'une telle thérapie sur des patients souffrant d'insuffisance cardiaque, d'évaluer l'efficacité de la thérapie afin d'en apprécier la pertinence, et dans l'affirmative la poursuivre en modifiant éventuellement les paramètres de fonctionnement du dispositif implanté.

Les paramètres spécifiques de la stimulation CRT sont représentés par une "configuration de stimulation", constituée par la combinaison des caractéristiques relatives aux "sites de stimulation" et des caractéristiques relatives à la "séquence de stimulation". Les "sites de stimulation" se réfèrent à l'emplacement physique des électrodes intracardiaques par rapport au tissu du myocarde. Ces sites peuvent être choisis au moment de l'implantation par un positionnement approprié des électrodes. Dans le cas des prothèses dites "multisite" où le dispositif comporte plusieurs électrodes placées dans une même cavité, la modification du site de stimulation dans cette cavité est également possible par une commutation interne du dispositif. La notion de "séquence de stimulation", quant à elle, se réfère d'une part à l'ordre suivant lequel les impulsions de stimulation sont appliquées au coeur (oreillette/ventricule gauche/ventricule droit), et d'autre part aux intervalles de temps séparant l'application de ces impulsions successives. Ici encore, la séquence de stimulation est paramétrée à l'implantation, mais peut être modifiée par la suite au moyen de commutations internes appropriées du dispositif et par ajustement des paramètres de séquencement des impulsions.

Il est nécessaire d'évaluer régulièrement la pertinence de la configuration de stimulation, car celle-ci conditionne l'efficacité de la thérapie par stimulation biventriculaire. De plus, les effets bénéfiques procurés par cette thérapie peuvent conduire, à terme, à réévaluer la configuration primitive pour éventuellement modifier le choix des sites et/ou le paramétrage de la séquence de stimulation.

L'une des pratiques de référence utilisées pour optimiser les paramètres de stimulation consiste à estimer les délais caractéristiques de la systole, en particulier le temps d'ouverture de la valve aortique, par une évaluation échographique. Cette procédure, qui doit être mise en oeuvre en milieu hospitalier et par un personnel qualifié, est longue et coûteuse et ne peut pas être appliquée aussi souvent que cela serait utile ou nécessaire sans interférer avec la vie quotidienne du patient.

Une autre solution, proposée par le EP 0 108 446 A1 précité, consiste à évaluer le degré de synchronisation des contractions des ventricules par une mesure de bioimpédance intracardiaque, cette donnée étant en effet représentative du débit cardiaque et donc de la fraction d'éjection, considérée comme le paramètre hémodynamique de référence.

La présente invention est basée sur une autre approche de la stimulation biventriculaire, mettant en oeuvre une analyse de l'accélération endocardiaque (ci-après désignée "EA").

En effet, les études cliniques qui ont été menées indiquent que l'accélération endocardiaque est un paramètre permettant de fournir des informations très complètes sur l'état fonctionnel du myocarde, aussi bien dans le cas d'un fonctionnement normal que d'un fonctionnement déficient : l'accélération endocardiaque, qui est mesurée par un accéléromètre directement en contact avec le muscle cardiaque (généralement, mais non exclusivement, à l'apex ventriculaire droit, parfois dans l'oreillette droite), reflète en effet très précisément et en temps réel les phénomènes concourant au fonctionnement mécanique du coeur.

Plus précisément, le EP 0 515 319 A1 (Sorin Biomedica Cardio SpA) enseigne la manière de recueillir un signal d'accélération endocardiaque au moyen d'une sonde endocavitaire pourvue d'une électrode distale de stimulation implantée au fond du ventricule et intégrant un microaccéléromètre permettant de mesurer l'accélération endocardiaque.

Le signal d'accélération endocardiaque recueilli au cours d'un cycle cardiaque forme deux composantes principales, correspondant aux deux bruits majeurs du coeur (sons S1 et S2 du phonocardiogramme) qu'il est possible de reconnaître dans chaque cycle cardiaque :

- la première composante d'accélération endocardiaque ("EA1"), dont les variations d'amplitude sont étroitement liées aux variations de la pression dans le ventricule (l'amplitude maximale crête-à-crête de cette composante EA1, appelée PEA1, étant plus précisément corrélée au maximum positif de la variation de pression dP/dt dans le ventricule gauche) et peuvent donc constituer un paramètre représentatif de la contractilité du myocarde, elle-même liée au niveau d'activité du système sympathique ;

- la seconde composante d'accélération endocardiaque ("EA2") qui, quant à elle, survient pendant la phase de relaxation ventriculaire iso-volumique. Cette seconde composante est produite, principalement, par la fermeture des valves aortiques et pulmonaires.

Le signal EA contient parfois une ou deux autres composantes, appelées EA3 et EA4, correspondant aux sons S3 et S4 du phonocardiogramme. Ces bruits témoignent le plus souvent d'une insuffisance cardiaque (EA3 étant a priori dû aux vibrations des parois du myocarde pendant le remplissage rapide, et EA4 étant a priori dû à la contraction auriculaire).

Le terme "composante EAx" se référera par la suite indifféremment à l'une de ces quatre composantes, en particulier (mais de façon non limitative) la composante EA1 ou la composante EA2.

Le EP 0 655 260 A1 (Sorin Biomedica Cardio SpA) décrit une manière de traiter le signal d'accélération endocavitaire délivré par le capteur situé en bout de sonde pour en dériver deux valeurs liées à ces pics d'accélération endocardiaque respectifs, valeurs utiles notamment pour la détection de troubles cardiaques et le déclenchement ou non d'une thérapie de défibrillation.

Le EP 1 736 203 A1 (ELA Medical) décrit une application spécifique aux implants à stimulation biventriculaire, consistant à utiliser les paramètres liés à l'accélération endocardiaque pour déterminer une configuration de stimulation optimale pour le patient, au moment de l'implantation ou ultérieurement. Diverses mesures sont effectuées pour caractériser le signal EA, et sont combinées pour donner un indice de performance composite, le choix de la configuration de stimulation finale étant celui qui maximise cet indice de performance.

Le US 7 139 609 B1 décrit également un dispositif implanté capable d'assurer un suivi de la fonction cardiaque à partir d'un signal d'accélération endocardiaque, pour optimiser le fonctionnement général du stimulateur ou appliquer une thérapie de resynchronisation ventriculaire. Chaque cycle cardiaque est analysé de manière à identifier les deux bruits majeurs et analyser le signal pour en déduire des paramètres tels que dP/dt, volume d'éjection, etc.

Le EP 1 741 387 A1 décrit une technique de diagnostic consistant à mesurer l'amplitude crête-à-crête de EA1 (appelée PEA1) et/ou l'amplitude crête-à-crête de EA2 (appelée PEA2) au cours de plusieurs cycles successifs, et analyser les variations de cette amplitude pour détecter une situation d'apnée ou d'hypopnée et délivrer une alerte appropriée.

On notera que, bien que dans la présente description on fasse principalement référence à l'analyse d'un signal EA délivré par un capteur implanté (typiquement, un capteur placé sur une sonde endocavitaire), l'invention est également applicable à une analyse opérée à partir d'un signal EA externe obtenu de manière non invasive. Un tel signal EA externe peut être par exemple issu d'un capteur fixé sur la poitrine du patient au niveau du sternum, le signal ECG étant simultanément recueilli au moyen d'électrodes externes et enregistré. Ici et dans la suite, lorsque l'on parlera de "signal EA", ce terme sera entendu aussi bien au sens de signal EA externe, obtenu de manière non invasive, que de signal EA endocavitaire, obtenu par un capteur d'accélération monté sur une sonde implantée en contact direct avec le myocarde du patient (le dispositif disposant dans ce cas d'un signal d'électrogramme EGM enregistré simultanément).

L'un des buts de l'invention est de proposer un dispositif mettant en oeuvre une technique perfectionnée d'analyse du signal EA afin de pouvoir en extraire un certain nombre d'informations significatives, représentatives de l'activité mécanique et hémodynamique du coeur du patient, avec notamment élimination de l'influence des variations cycle-à-cycle du signal EA, variations qui sont susceptibles de fausser les résultats délivrés par l'algorithme chargé d'analyser ce signal EA.

Un autre but de la présente invention est de proposer un dispositif mettant en oeuvre une technique d'analyse du signal EA qui tienne également compte de la spécificité de chacune des composantes EAx, notamment des deux composantes EA1 et EA2, considérées séparément.

Il convient à ce stade de remarquer que l'invention n'est pas limitée à l'analyse des seules composantes EA1 et EA2 (que ces composantes soient considérées isolément ou conjointement) et que, en variante ou en complément, elle est applicable de façon comparable à l'analyse des composantes EA3 et/ou EA4 associées aux sons S3 et S4 du phonocardiogramme. Pour la clarté, on ne fera référence qu'aux composantes "EA1" et "EA2", mais tout ce qui sera dit à propos de ces composantes "EA1" et "EA2" doit être considéré comme applicable *mutatis mutandis* aux composantes EA3 et/ou EA4.

De façon générale, l'un des buts de l'invention est de proposer une nouvelle technique d'analyse du signal EA permettant d'augmenter la spécificité et la pertinence des résultats fournis par l'algorithme d'analyse du signal EA, tout particulièrement en vue d'obtenir des caractéristiques corrélées à des marqueurs temporels de la systole ("timings" du cycle cardiaque) et à d'autres indices de performance hémodynamique du myocarde.

Il s'agit de pouvoir suivre l'évolution des caractéristiques ainsi extraites, battement après battement, de manière à pouvoir estimer à chaque instant les performances hémodynamiques du coeur et donc délivrer une thérapie de resynchronisation ventriculaire optimale pour le patient. Cette estimation doit notamment pouvoir tenir compte de l'évolution de la configuration de stimulation optimale au cours du temps et du fait que celle-ci peut être différente au repos et à l'effort (actuellement la configuration de stimulation est optimisée au repos seulement).

Outre l'optimisation automatique de la thérapie, l'analyse du signal EA peut fournir des indications précieuses notamment pour :

- le guidage de la sonde lors de l'implantation, à partir de certaines caractéristiques EA présélectionnées ;
- la localisation d'un site de stimulation optimale (position de la sonde gauche ou droite) ;
- le diagnostic de l'état de santé du patient, par un suivi permanent du risque d'insuffisance cardiaque ;
- la discrimination des arythmies ;
- l'impact d'événements tels qu'apnée, arythmie, etc. sur l'hémodynamique du patient.

L'invention propose à cet effet un dispositif d'analyse du type général défini dans le préambule de la revendication 1, les éléments spécifiques de l'invention étant énoncés dans la partie caractérisante.

Les sous-revendications visent des mises en oeuvre particulières avantageuses.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 est une série de trois chronogrammes illustrant différents signaux caractérisant l'activité cardiaque au cours d'un cycle donné.

La Figure 2 est un organigramme général des différentes étapes de l'analyse du signal EA opérée par le dispositif de l'invention.

La Figure 3 illustre le découpage d'un enregistrement continu en une série de cycles cardiaques successifs.

La Figure 4 illustre l'extraction des deux composantes EA1 et EA2 sur un sous-signal EA.

La Figure 5 illustre un exemple de calcul de fonctions d'intercorrélation entre deux sous-signaux, pour recaler les composantes des différents cycles successifs.

La Figure 6 illustre la génération du cycle moyen à partir des données prétraitées pour chacune des deux composantes EA1 et EA2.

La Figure 7 illustre deux exemples de composantes EA1 pour deux signaux différents, l'un présentant une faible valeur de contraste et l'autre une forte valeur de contraste.

Les Figures 8, 9a et 9b illustrent l'étape de traitement par calcul d'une enveloppe énergétique de Shannon.

La Figure 10 illustre l'étape de traitement par application d'un modèle auto-régressif estimé de manière récursive.

La figure 11 illustre l'étape de calcul d'une valeur de rapport signal/bruit, appliquée à la composante EA2.

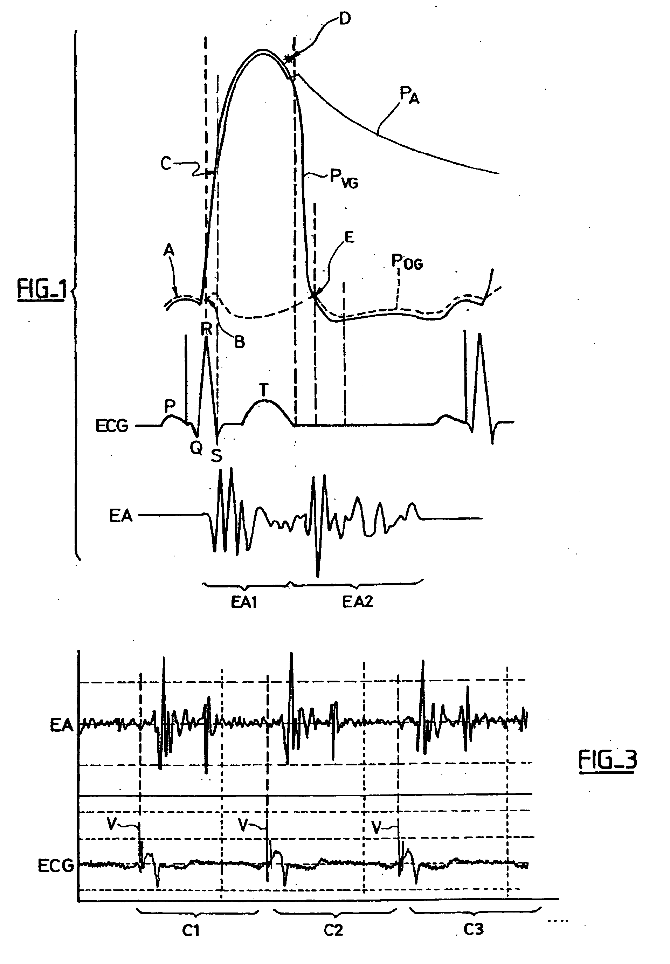

La Figure 1 illustre les différents signaux caractérisant l'activité du coeur au cours d'un cycle cardiaque, avec : le profil des pressions intracardiaques ($P_A$, $P_{VG}$ et $P_{OG}$), un relevé d'électrocardiogramme de surface (ECG), et les variations du signal d'accélération endocardiaque (EA).

La caractéristique $P_A$ illustre les variations de la pression aortique, $P_{VG}$ celles du ventricule gauche et $P_{OG}$ celles dans l'oreillette gauche. Les points A à E correspondent aux différentes phases suivantes : A contraction de l'oreillette gauche, B fermeture de la valvule mitrale, C ouverture de la valvule aortique, D fermeture de la valvule aortique, E ouverture de la valvule mitrale.

Le signal ECG présente successivement l'onde P correspondant à la dépolarisation des oreillettes, le complexe QRS correspondant à la dépolarisation des ventricules et l'onde T de repolarisation ventriculaire.

Le signal d'accélération endocardiaque EA, quant à lui, peut se décomposer en deux composantes successives : la composante EA1, qui débute à la suite du complexe QRS, est engendrée par une combinaison de la fermeture des valves auriculo-ventriculaires, de l'ouverture des valves semi-lunaires et de la contraction du ventricule gauche ; la composante EA2 qui lui fait suite, quant à elle, accompagne la fin de la systole ventriculaire et elle est générée par la fermeture des valves semi-lunaires.

Ces deux composantes EA1 et EA2 correspondent classiquement, comme on l'a indiqué plus haut, aux deux principaux bruits cardiaques.

Essentiellement, la présente invention propose d'extraire du signal d'accélération, et plus précisément des deux composantes EA1 et EA2 (et/ou EA3 et EA4) par un traitement spécifique de chacune d'entre elles, des caractéristiques corrélées à des intervalles de temps de la systole et à d'autres indices de performance hémodynamique du myocarde.

Comme indiqué plus haut, bien que l'on décrive une mise en oeuvre de l'invention basée sur une analyse des composantes EA1 et EA2, l'invention est aussi bien applicable à l'analyse des composantes EA3 et EA4 associées aux sons S3 et S4 du phonocardiogramme, et tout ce qui sera dit ci-après en référence aux composantes "EA1" ou "EA2" est transposable *mutatis mutandis* aux composantes EA3 et EA4.

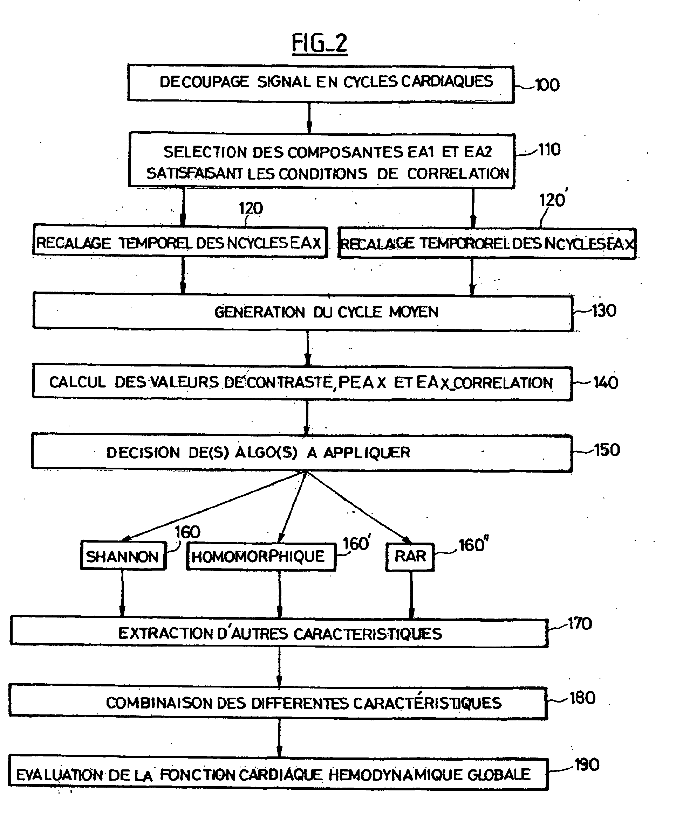

La séquence générale du procédé d'analyse selon l'invention est explicitée par l'organigramme de la Figure 2.

Cette analyse du signal EA nécessite l'acquisition d'un minimum de N battements cardiaques successifs. L'analyse peut être opérée :

- soit, dans un mode "monitoring", sur une fenêtre d'analyse glissante de N battements (N ≥ 1), pour suivre l'évolution temporelle des différentes caractéristiques du signal EA (dans ce cas N = 5, typiquement),
- soit, dans un mode "balayage des configurations", sur une pluralité d'enregistrements de N battements successifs acquis avec différentes configurations de stimulation dans des conditions stables, de manière à déterminer la configuration de stimulation optimale en fonction d'un certain nombre de critères prédéterminés (dans ce cas N = 5, typiquement).

L'acquisition du signal EA peut se faire, comme exposé plus haut, aussi bien au moyen d'une prothèse implantée pourvue d'un capteur approprié, par exemple un capteur d'accélération en bout de sonde, qu'avec un capteur externe appliqué sur le thorax du patient et recueillant les bruits du coeur, par exemple pendant un test d'effort.

La première étape du processus, référencée 100 sur la Figure 2, consiste à opérer un découpage du signal EA recueilli en continu sur N battements successifs et préalablement filtré par un filtre passe-bande, de manière à individualiser dans ce signal continu les différents cycles cardiaques successifs.

Comme illustré Figure 3, il s'agit de définir les cycles cardiaques successifs C1, C2, C3 ... du signal EA continu en déterminant des marqueurs de début de cycle permettant d'individualiser ces cycles cardiaques, de manière à produire une série de sous-signaux EA correspondant chacun à une durée d'un seul cycle cardiaque :

- dans le cas d'un signal EA endocavitaire, les marqueurs temporels de début de cycle sont fournis par l'implant lui-même, qui garde en mémoire les instants de stimulation V (comme illustré Fig. 3) ou les instants de détection de l'onde R, selon le mode de fonctionnement ;
- dans le cas d'un signal EA externe, les marqueurs temporels de début de cycle cardiaque sont fournis par un algorithme de détection des pics de stimulation ou des complexes QRS du signal ECG, signal recueilli par ailleurs au moyen d'électrodes externes.

Avantageusement, l'analyse met également en oeuvre un algorithme de détection d'extrasystoles, en lui-même connu, permettant dans un tel cas d'éliminer les cycles cardiaques affectés par les extrasystoles détectées, à savoir : le cycle précédant l'extrasystole, le cycle incluant l'extrasystole elle-même, ainsi que le cycle suivant l'extrasystole.

Les divers sous-signaux EA correspondant chacun à une durée d'un cycle cardiaque ainsi individualisé sont ensuite analysés de manière à déterminer une longueur de cycle moyenne, ou de préférence médiane, sur les N battements successifs.

L'algorithme opère alors un redécoupage du signal EA continu, de manière à définir des sous-signaux EA réajustés sur cette même longueur médiane commune. Cette technique permet en particulier d'éviter les situations dans lesquelles un pic de stimulation n'aurait pas été détecté, ce qui aurait donné un sous-signal EA de durée environ double de celle de la durée réelle.

Après cet ajustement de durée, on obtient une matrice composée de N sous-signaux EA, ayant tous une même durée, durée égale à la durée médiane calculée.

L'étape suivante, correspondant au bloc 110 de la Figure 2, consiste à isoler les deux composantes EA1 et EA2 dans chaque sous-signal EA, de manière à pouvoir ensuite effectuer un certain nombre de traitements de manière distincte pour chacune de ces composantes EA1 et EA2.

L'extraction des deux composantes EA1 et EA2 est opérée par une technique de corrélation appliquée aux N sous-signaux EA obtenus de la manière indiquée plus haut.

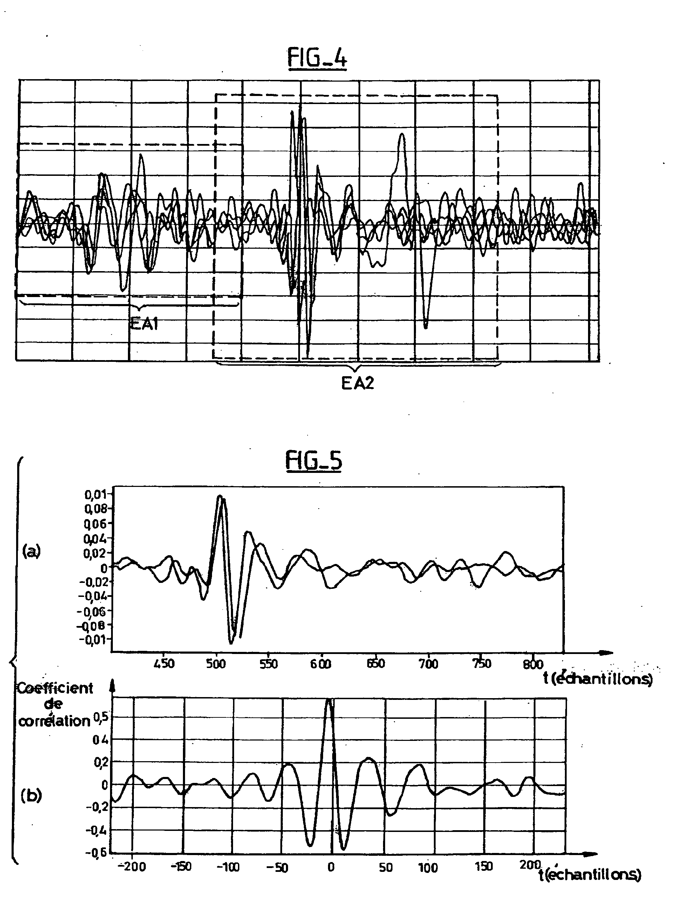

La Figure 4 illustre les deux fenêtres de signal utile EA1 et EA2, obtenues en exploitant la reproductibilité des deux composantes EA1 et EA2 sur les N sous-signaux EA. La durée de chacune de ces deux fenêtres peut être une durée fixe, ou éventuellement une durée variable, notamment une durée fonction d'un pourcentage de l'intervalle RR de manière à permettre une meilleure adaptation au cas des rythmes rapides. On notera que ces deux fenêtres peuvent être partiellement chevauchantes.

À partir de la matrice des N sous-signaux EA, on obtient ainsi deux matrices indépendantes contenant respectivement les composantes EA1 et

EA2 des N cycles cardiaques analysés.

L'étape suivante, référencée 120 ou 120' sur la Figure 2, consiste à opérer un recalage temporel des N cycles, distinctement et parallèlement pour chacune des deux composantes EA1 et EA2.

Une première technique consiste, pour chaque paire de sous-signaux de la matrice, à rechercher le maximum de la fonction d'intercorrélation normalisée, fonction qui variera entre 1 (dans le cas de deux vecteurs parfaitement corrélés) et 0 (dans le cas de deux vecteurs non corrélés) :

$$\Gamma_{i,j}(\tau) = \frac{\sum_{t=0}^{Nsamples-\tau-1}\left(ea\_cycles_i(t+\tau)-\mu_{ea\_cycles_i}\right)\cdot\left(ea\_cycles_j(t)-\mu_{ea\_cycles_j}\right)}{\sqrt{\sum_{t=0}^{Nsamples-1}\left(ea\_cycles_i(t)-\mu_{ea\_cycles_i}\right)^2 \cdot \sum_{t=0}^{Nsamples-1}\left(ea\_cycles_j(t)-\mu_{ea\_cycles_j}\right)^2}}$$

Dans cette expression $i$ ou $j$ = 1 ou 2, $ea\_cycles_1(t)$ et $ea\_cycles_2(t)$ représentant les termes des matrices des N sous-signaux de la composante respective considérée, EA1 ou EA2 (le même traitement est opéré pour chacune des deux composantes).

Le résultat est illustré Figure 5 : la Figure 5a illustre la paire de sous-signaux analysés et la Figure 5b la fonction d'intercorrélation correspondante. Dans l'exemple de la Figure 5, le maximum de la fonction d'inter-corrélation se situe à $\tau_{i,j}$ = - 5 échantillons, avec une valeur de pic de 0,78.

Cela signifie qu'en retardant $ea\_cycles_1(t)$ de 5 échantillons, $ea\_cycles_1(t)$ et $ea\_cycles_2(t)$ sont corrélés avec $r = 0.78$.

On construit alors deux tableaux : l'un contenant des coefficients de corrélation $r_{i,j}$ des différentes paires, l'autre contenant les délais $\tau_{i,j}$ permettant de recaler les différents sous-signaux $ea\_cycles_i(t)$ les uns par rapport aux autres.

On repère ensuite quel est celui des cycles le mieux corrélé aux autres $ea\_cycles_i(t)$ ($i \neq j$), en calculant pour chaque $i$ le coefficient de corrélation moyen avec les autres sous-signaux.

Le sous-signal de référence sera celui présentant le coefficient de corrélation moyen maximum : $reference\_cycle\_ind\_EAx$. Puis on retient les sous-signaux $ea\_cycles_j(t)$ (avec $j \neq reference\_cycle\_ind\_EA_x$) qui répondent aux deux critères suivants :

$$r_{reference\_cycle\_ind\_EAx,j} > \text{seuil de corrélation EA}_x, \text{ et}$$

$$\left|\tau_{reference\_cycle\_ind\_EAx,j}\right| < \text{seuil de délai de corrélation.}$$

Sur les N sous-signaux d'origine, il reste N' cycles EA$_x$ après cette étape de sélection. En gardant uniquement les indices $j$ des cycles sélectionnés, on définit les valeurs $EAx\_corrélation$ = moyenne ($r_{reference\_cycle\_ind\_EAx,j}$) (avec j$\neq$ $reference\_cycle\_ind\_EA_x$)

Une variante de mise en oeuvre de ce procédé, moins exigeante en temps de calcul mais moins robuste, consiste à fixer le cycle de référence avant de calculer les corrélations. Ce choix peut être manuel ou aléatoire, ou fixe (par exemple le premier cycle détecté), ce qui diminue de façon importante le nombre de combinaisons à analyser, le reste de la méthode étant identique.

Une autre variante de mise en oeuvre de ce procédé, également moins exigeante en temps de calcul mais moins robuste, consiste à recaler les N cycles par rapport à l'instant $t_{PEAx}$ d'arrivée de l'amplitude pic à pic de la composante $EA_x$ de chaque cycle :

$$t_{PEAx} = 0,5 \times [\ t\_min(EAx) + t\_max(EAx)].$$

Les décalages $\tau_{i,j}$ entre chaque paire de cycles sont calculés de manière à ce que ces instants d'amplitude pic à pic soient synchrones. Il suffit de calculer un coefficient de corrélation uniquement pour cette valeur de décalage, ce qui diminue de façon importante le nombre d'opérations à réaliser, le reste de la méthode étant identique.

Une autre variante encore, particulière avantageuse en termes de ressources logicielles, consiste à opérer une détection des pics conformément à un algorithme en métalangage tel que celui-ci :

Pour chaque cycle EAx :

Localiser le pic du maximum, en valeur absolue, à l'intérieur de la fenêtre ;
Ouvrir une fenêtre autour de ce pic (commençant par exemple 100ms avant le maximum et se terminant 100ms après, ce paramètre étant éventuellement ajustable) ;

Localiser deux extrema locaux à l'intérieur de cette fenêtre, éventuellement avec ajout d'une condition supplémentaire sur la dérivée seconde (en testant la valeur de l'échantillon i avec celle des échantillons i+2 et i-2) pour discriminer un type particulier d'extremum, et conserver les trois extrema présentant les amplitudes absolues les plus grandes ; s'il n'y a pas d'autre extremum que le pic, ne conserver que le pic ;

Ensuite, entre un EAx de référence et chacun des autres EAx de la fenêtre d'analyse :

calculer toutes les distances inter-extrema possibles ;

sélectionner les deux pics les plus proches, qui seront considérés comme des pics de référence ;

calculer l'intervalle temporel rentre ces deux pics de référence ;

recaler de r le cycle cardiaque courant par rapport au cycle de référence ;

calculer enfin le coefficient de corrélation normalisé $r$, pour cette valeur

de $\tau$ uniquement.

On notera enfin qu'il est possible de combiner entre elles les quatre méthodes différentes de recalage décrites ci-dessus.

Dans le cas d'une analyse glissante (fonction "monitoring") il suffira d'éliminer le premier cycle conforme, et d'opérer l'analyse avec le prochain cycle détecté. Si ce prochain cycle satisfait les conditions de corrélation, ce cycle est ajouté au cycle conforme, sinon l'analyse est opérée sur le cycle suivant. Si toutefois il n'est pas possible de vérifier ces conditions après cinq cycles successifs, on considérera que la composante correspondante EA1 ou EA2 n'est pas reproductible d'un cycle à l'autre. Si, en mode "monitoring", le nombre de battements désirés pour la fenêtre d'analyse glissante vaut 1, cette étape de prétraitement ne sera pas appliquée, et l'on passera directement au bloc 140 de la Figure 2.

À ce stade, il est possible d'appliquer un algorithme réjecteur d'artefacts.

Cet algorithme peut notamment se baser sur le calcul de l'une des quantités suivantes, au choix :

$$\mu^i_{EAx\_window} = \frac{1}{N_{EAx\_window}} \cdot \sum_{t \in EAx\_window} \left( ea\_cycles_i(t) \right)$$

$$\sigma^i_{EAx\_window} = \sqrt{\frac{1}{N_{EAx\_window}} \cdot \sum_{t \in EAx\_window} \left( ea\_cycles_i(t) - \mu^i_{EAx\_window} \right)^2}$$

$$B_i = \sum_{t \in EAx\_window} (ea\_cycles_i(t))^2$$

$N_{EAx\_window}$ étant le nombre d'échantillons dans la fenêtre $EAx\_window$.

L'algorithme calcule ensuite la moyenne $\mu_{CRIT\_ARTEFACT}$ et l'écart-type $\sigma_{CRIT\_ARTEFACT}$ de la grandeur choisie, sur l'ensemble des N cycles EAx. On peut alors choisir de ne sélectionner que les composantes i telles que $|CRIT\_ARTEFACT(i) - \mu_{CRIT\_ARTEFACT}| < \alpha \cdot \sigma_{CRIT\_ARTEFACT}$, $\alpha$ étant un coefficient paramétrable (typiquement égal à 2).

L'étape suivante, correspondant au bloc 130 de la Figure 2, consiste à définir un cycle moyen à partir des composantes EA1 et EA2, prétraitées séparément de la manière indiquée plus haut.

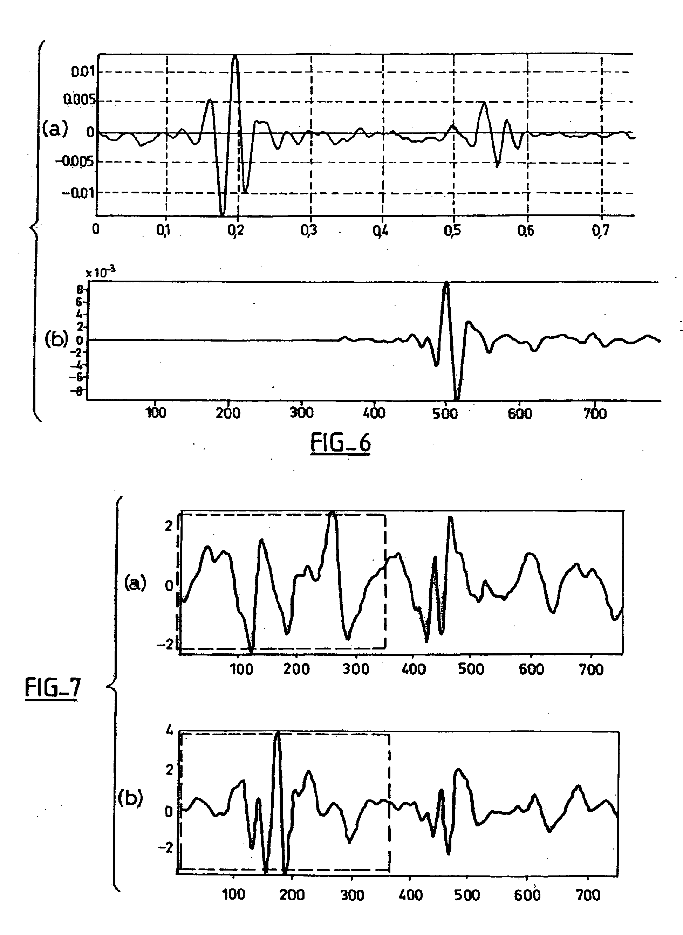

A cet effet, les sous-signaux EA1 et EA2 de chaque cycle sont recalés par rapport au sous-signal de référence $reference\_cycle\_ind\_EA_x$. On commence par centrer les valeurs $\tau_{reference\_cycle\_ind\_EAx,j}$ calculées à l'étape précédent en leur soustrayant la valeur moyenne des $\tau_{reference\_cycle\_ind\_EAx,j}$ sur l'ensemble des j (y compris le cycle de référence). On utilise ensuite ces nouvelles valeurs de $\tau_{reference\_cycle\_ind\_EAx,j}$ pour recaler les sous-signaux EA1 et EA2 par rapport au sous-signal de référence $reference\_cycle\_ind\_EA_x$. Une fois les sous-signaux recalés, il suffit de calculer une composante EA1 ou EA2 moyenne et de former ainsi le signal EA moyen par combinaison des deux composantes. Ce signal EA moyen est illustré Figure 6a.

Si l'une des deux composantes EA1 ou EA2 n'a pas satisfait une condition *Ncycles EAx > NcyclesEAx_min*, un cycle global moyen est tout de même formé, en remplaçant la composante en question par une valeur nulle. Cet cas est illustré Figure 6b, dans le cas où la composante EA1 ne répondrait pas aux critères.

À ce stade, une fois que le cycle moyen a été déterminé, il est possible d'y appliquer éventuellement une pondération afin d'atténuer certaines composantes dont on connaît la position relative dans le cycle cardiaque. Par exemple, on sait que la composante EA4 arrive entre le début de l'activité électrique auriculaire (l'onde P dans le cas d'un ECG, ou la détection de la dépolarisation sur la sonde auriculaire dans le cas d'un dispositif implantable) et le début de la composante EA1. Si l'on veut éviter que la composante EA4 ne perturbe les étapes suivantes du traitement, on pourra appliquer une fonction de pondération construite à partir d'une fenêtre de forme, durée et position choisies. Cette fenêtre de pondération peut par exemple être une fenêtre de Hamming de durée 75 ms avec une position temporelle du minimum de la fonction de pondération à 0 ms.

L'étape suivante, correspondant au bloc 140 de la Figure 2, consiste à calculer des valeurs de contraste et d'entropie pour chacune des composantes EA1 et EA2.

La valeur de contraste est calculée en déterminant l'amplitude pic à pic du cycle EA moyen sur la fenêtre considérée. Le contraste de la composante EAx est donné par une formule telle que :

$$contrast\_EAx = \frac{PEAx}{2 \cdot \sigma_{EAx\_window}}$$

Une valeur de contraste élevée signifie que la composante utile EAx est localisée dans le temps. Au contraire, une valeur de contraste plus faible correspond à un étalement beaucoup plus important de la composante EAx. Ces deux situations sont illustrées par les exemples des Figure 7a (faible valeur de contraste) et 7b (valeur de contraste correcte).

On définit sur les mêmes fenêtres d'analyse une valeur d'entropie donnée par une formule telle que :

$$entropy\_EAx = -\sum_{EAx\_window}\left(average\_ea\_cycles_i(t) \cdot \log 10(average\_ea\_cycles_i(t))\right)$$

Cette quantité reflète le "degré d'ordre" du signal : si le signal est proche d'un bruit blanc l'entropie sera élevée, si au contraire il est "ordonné", l'entropie sera plus faible.

Il est à ce stade possible d'évaluer un indice de morphologie des composantes EA1 et/ou EA2 pouvant être corrélés à des indices de performances du coeur, ou pouvant servir à choisir telle ou telle version d'algorithme, mieux adaptée à une certaine morphologie. Par exemple, le dispositif détermine pour ce faire une courbe d'enveloppe énergétique par l'une des méthode décrites par la suite, puis calcule pour chaque composante EAx l'aire sous cette courbe : l'indice de morphologie est alors donné par l'inverse de cette aire.

Également à ce stade, il est possible d'opérer un filtrage passe-bas, passe-haut, passe-bande ou un filtrage adaptatif du signal EA moyen avant la génération de l'enveloppe, par exemple un filtrage passe-haut sur 128 échantillons à 25 Hz.

Il est possible d'utiliser à cet effet des filtres à réponse impulsionnelle finie (FIR) ou des filtres à réponse impulsionnelle infinie (IIR). On peut choisir de filtrer dans le sens du déroulement des échantillons uniquement, ou de filtrer dans ce sens puis de filtrer avec le même filtre la sortie de ce filtre dans le sens opposé, afin de compenser le déphasage induit par le filtrage.

Un filtrage adaptatif peut être effectué également sur le signal EA moyen avant tout autre traitement, par un filtre tel que :

$$y(nT) = \hat{n}_1(nT) = \sum_{k=0}^{p} w_{k,nT} x_2(nT - kT)$$

$y(nT)$ étant la sortie du filtre adaptatif,
$x_2(nT)$ étant le signal de référence, et

$w_{k,nT}$ étant les coefficients du filtre, qui seront appris de façon adaptative.

Un algorithme LMS (*Least Mean Squares*) peut être utilisé pour apprendre les coefficients du filtre de la façon suivante :

$$w_n = w_{n-1} + 2\mu\big(x_1(nT) - y(nT)\big)x_2(nT)$$

$x_1(nT)$ étant le signal à traiter, ici le signal EA moyen.

Ce filtrage adaptatif est particulièrement utile pour séparer la composante EA4 qui peut être superposée à la composante EA1, garantissant ainsi un meilleur traitement du signal EA1 filtré.

Dans ce dernier cas, le signal $x_2(nT)$ sera l'instant d'activation auriculaire (déterminé par traitement d'un signal ECG de surface ou d'un signal EGM), et l'apprentissage des coefficients du filtre pourra avoir une valeur de $\mu$ fonction du délai atrioventriculaire (délai AV) observé ou imposé par le dispositif, par exemple $\mu = 0$ pour des délais AV courts (pendant la superposition de EA4 sur EA1) et $\mu > 0$ pour des délais AV longs (quand EA4 et EA1 ne sont pas superposés).

L'étape de filtrage adaptatif pourra être également utilisée pour annuler le bruit 50/60 Hz dans le cas d'une acquisition externe (capteur thoracique).

Ce filtrage adaptatif peut être également utilisé pour isoler du signal la composante EA4 et permettre l'analyse ultérieure de cette dernière, notamment pour une utilisation à des fins diagnostiques (et de même pour la composante EA3).

Après cette étape (éventuelle) de filtrage, le signal EA moyen est ensuite soumis à une analyse globale, c'est-à-dire que cette analyse est opérée sur le signal EA moyen d'une durée correspondant à un cycle cardiaque, sans opérer de traitement différencié sur les deux composantes EA1 et

EA2 (à la différence des étapes de prétraitement exposées plus haut).

Avantageusement, l'algorithme peut mettre en oeuvre plusieurs méthodes d'analyse différentes (blocs 160, 160' et 160" sur la Figure 2), le choix de la méthode étant opéré (bloc 150 sur la Figure 2) en fonction des résultats du prétraitement que l'on vient de décrire.

Il est ainsi possible de privilégier une méthode d'analyse (ci-après "méthode de segmentation") au profit des autres, par exemple dans le cas où le prétraitement a révélé une bonne corrélation des composantes EA1 et/ou EA2. Il est également possible de combiner les résultats de plusieurs méthodes de segmentation, ou encore de ne déclencher aucune analyse, par exemple si l'on considère que l'une et/ou l'autre des composantes EA1 ou EA2 présente une allure trop chaotique (en fonction notamment des valeurs de contraste et d'entropie calculées à l'étape précédente).

Une première méthode de segmentation (bloc 160' sur la Figure 2), en elle-même connue, est basée sur le calcul d'une enveloppe énergétique, selon la formule générique suivante :

$$nrg(t) = \sum_{u=t-h\_nrg/2}^{t+h\_nrg/2} Fen\hat{e}tre(u) \times F\big(average\_ea\_cycle(u)\big)$$

*Fenêtre*(*u*) étant une fenêtre de pondération de largeur *h_nrg* (rectangle, fenêtre de Hamming ou autre fenêtre de pondération) pour lisser plus ou moins les effets de bords,

la fonction *F* appliquée aux échantillons *average_ea_cycle*(*u*) pouvant être une fonction quelconque (linéaire ou non-linéaire), par exemple :

- la transformée de Shannon $F_{shannon}(x) = -x^2 \cdot \ln(x^2)$,

- une fonction polynomiale $F_{poly\_n}(x) = \sum_{i=0}^{n} a_i \cdot x^i, n \in Z$, ou

- la fonction valeur absolue $F_{abs}(x) = |x|$.

Il est également possible, si nécessaire, de sous-échantillonner l'enveloppe d'énergie obtenue.

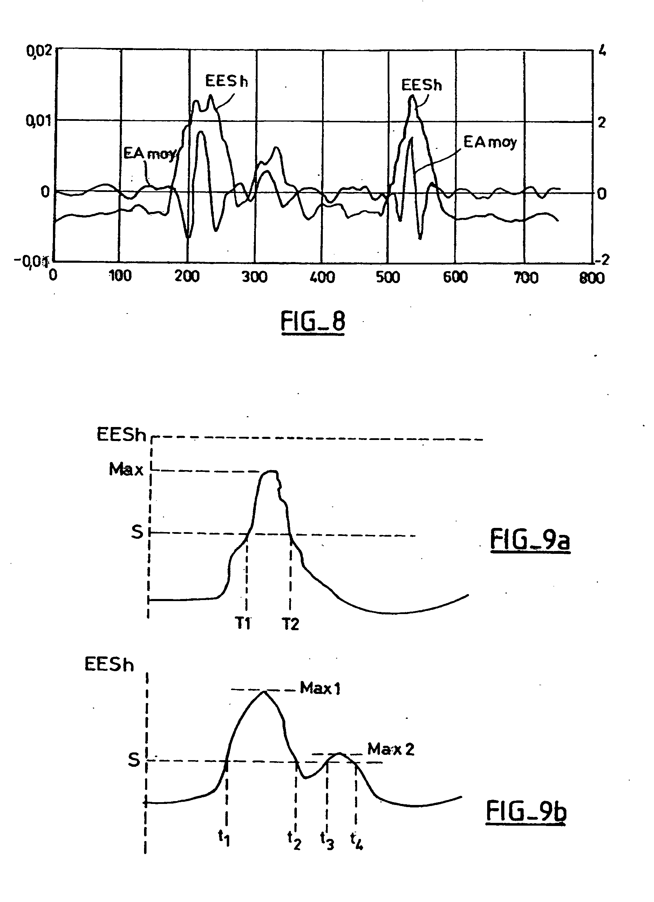

La Figure 8 illustre, sur un même chronogramme, le cycle EA moyen et l'enveloppe énergétique de Shannon EESh ainsi calculée.

Sur la fenêtre d'analyse EA1 ou EA2 considérée, l'algorithme recherche le maximum d'amplitude de l'enveloppe énergétique, et conserve sa valeur et les marqueurs temporels associés. Il recherche ensuite le premier échantillon de l'enveloppe énergétique précédant le maximum, et dont la valeur est égale à un seuil S donné (un pourcentage de ce maximum, ou une valeur particulière). Ceci permet de détecter le front montant de l'enveloppe et de définir un marqueur temporel T1, comme illustré Figure 9a.

De la même façon, pour la partie postérieure au maximum, le franchissement du seuil définit un deuxième marqueur temporel T2 de fin de l'enveloppe d'énergie du cycle EA moyen.

On notera que le seuillage peut prendre en compte d'autres paramètres et critères, notamment prendre en compte l'écart temporel entre T1 et T2, prévoir les cas de croisements multiples du seuil résultant de profils en "double bosse", etc.

Par exemple, on a illustré Figure 9b un cas où la courbe représentative de l'enveloppe franchit le seuil S quatre fois, en $t_1$, $t_2$, $t_3$ et $t_4$. Différentes approches sont possibles à l'égard d'une telle situation :

- retenir $t_1$ et $t_4$, les deux points extrêmes ;
- retenir $t_1$ et $t_2$, les points relatifs à la composante la plus énergétique (Max1 > Max2) ;
- retenir $t_3$ et $t_4$, les points relatifs à la composante la moins énergétique (Max2 < Max1) ;
- choisir quels points de début et de fin seront retenus, en fonction de seuils minima et maxima, par exemple :
  · $t_i$ et $t_j$ tels que $|t_j - t_i| > \Delta_{min}$ et $|t_j - t_i| = min\ (|t_x - t_y|)\ (x \neq y)$
  · $t_j$ et $t_i$ tels que $|t_j - t_i| < \Delta_{min}$ et $|t_j - t_i| = min\ (|t_x - t_y|)\ (x \neq y)$

Une deuxième méthode de segmentation (bloc 160' sur la Figure 2) est celle de l'enveloppe homomorphique.

Elle est mise en oeuvre avec application d'un filtre passe-bas permettant d'éliminer la composante de modulation en fréquence, composante typique des variations rapides que l'on cherche à éliminer. La composante en modulation d'amplitude obtenue après ce filtrage, appelée "envelogramme homomorphique" est traitée de manière comparable à la méthode précédente, pour déterminer des marqueurs T1 de début et T2 de fin de l'enveloppe d'énergie du cycle EA moyen.

Une troisième technique de segmentation, (bloc 160" su la Figure 2) consiste à appliquer un modèle autorégressif récursif (RAR).

L'idée est d'estimer la fréquence fondamentale du signal à chaque instant du signal EA, en analysant la phase des pôles d'un modèle autorégressif estimé de manière récursive (avec un critère d'élimination d'erreur RLS, *Recursive Least Square*, moindres carrés récursifs) ; cet algorithme est également connu sous le nom de "*forgetting factor approach*".

À cet effet, pour chaque échantillon du signal d'entrée l'algorithme estime un modèle autorégressif d'ordre 2 en considérant un certain segment de signal précédant cet échantillon (segment de largeur réglable par le paramètre "*forgetting factor*"), selon la formule suivante, où x est le vecteur signal d'entrée et où N = 2 dans le cas présent :

$$x[n] = -\sum_{k=1}^{N} a_k x[n-k] + v[n]$$

Le premier terme correspond à la prédiction linéaire, et le second à l'erreur de prédiction :

$$\overset{\cdots}{y} = \sum w_k x_k + \varepsilon$$

Cette équation peut être mise sous la forme d'un modèle auto-régressif :

$$H(z) = \frac{1}{1 + \sum_{k=1}^{N} a_k z^{-k}} \quad \blacktriangleright \quad P(\omega) = \frac{\sigma_v^2}{\left| 1 + \sum_{k=1}^{N} a_k e^{-j\omega k} \right|^2}$$

Il est possible de calculer les pôles du modèle par résolution d'une équation de second degré. En calculant la phase de ces pôles, on obtient une estimation de la fréquence instantanée du signal d'entrée :

$$freq(t) = \frac{phase(pôles) \cdot fs}{2\pi}$$

Le "*forgetting factor*" *rar_ff* est un paramètre très important, qui permet de régler la sensibilité de l'algorithme aux changements abrupts de fréquence, et donc au bruit. Ce paramètre peut également être exprimé par la notion de "*memory horizon*" :

$$rar\_ff = \frac{1}{1 - rar\_memory\_horizon \cdot fs}$$

Un paramètre "*memory horizon*" long indique une faible sensibilité au bruit, avec un temps de réponse de l'estimation long. Inversement, un paramètre court indique une forte sensibilité au bruit avec un temps de réponse court.
On notera que, bien que la méthode RAR soit en elle-même connue, elle a été perfectionnée, dans le cadre de la présente invention, par :

- un prétraitement supplémentaire, en ajoutant au signal d'entrée un bruit blanc de variance paramétrable, et
- un filtrage médian sur un certain nombre d'échantillons du signal de fréquence obtenu.

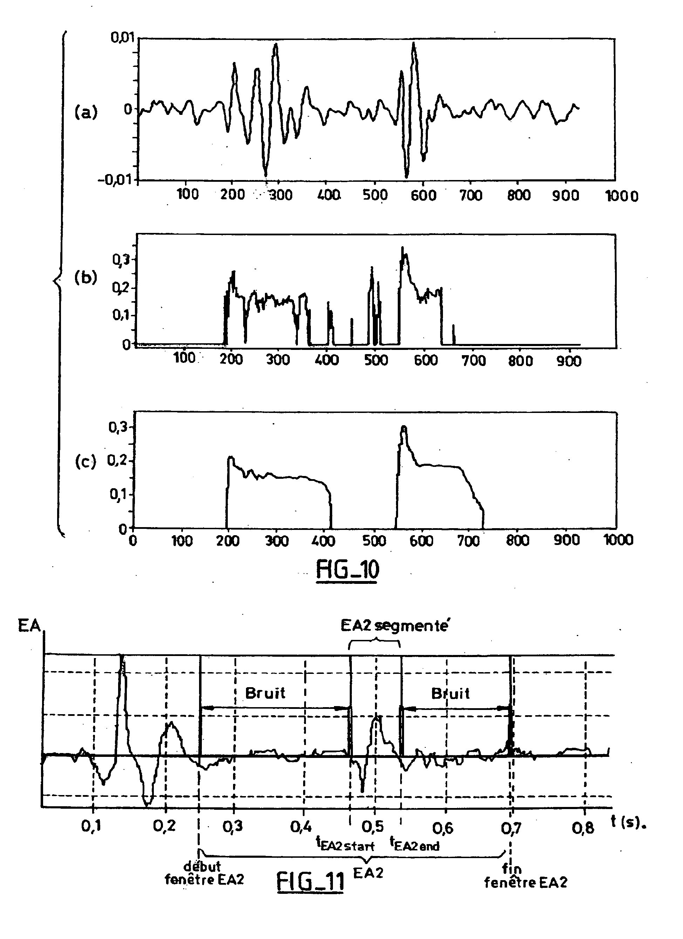

Ceci permet à l'algorithme de se caler uniquement sur les périodes utiles, comme le montre la Figure 10 : en 10a on a illustré le signal EA moyen objet du traitement, sur la Figure 10b le signal de fréquence estimée brut, et sur la Figure 10c le signal filtré médian finalement obtenu.
Il suffit de seuiller ce dernier signal par un seuil fixe pour déterminer les marqueurs T1 et T2 correspondant aux fronts montants et descendants de chacune des composantes EA1 et EA2.
Une fois les marqueurs temporels extraits du cycle moyen par l'une et/ou l'autre des différentes méthodes de segmentation exposées plus haut (blocs 160, 160' et 160"), l'étape facultative suivante (bloc 170 sur la Fig. 2) permet de revenir à des marqueurs temporels cycle à cycle, grâce aux valeurs des décalages calculés à l'étape 130.
En effet, les sous-signaux sélectionnés à cette étape 130 étaient décalés par rapport au sous-signal de référence. On peut donc calculer chaque instant de début $t\_EAx\_start\_xxx(j)$ (avec $j$ = 1 ... $NcyclesEAx$) en appliquant la formule suivante :

$$t\_EAx\_start_j = t\_EAx\_start\_av - \tau_{reference\_cycle\_ind\_EAx, i}.$$

On peut ainsi calculer une valeur moyenne des marqueurs temporels $\mu_{t\_EAx\_start}$, ainsi qu'un écart-type $\sigma_{t\_EAx\_start}$ pour avoir une idée de la variabilité cycle à cycle de ces marqueurs temporels sur $NcyclesEAx$ battements (en faisant l'hypothèse qu'en mode "balayage des configurations", le signal EA est stationnaire et que l'on peut ainsi se permettre d'approximer l'écart-type de ces marqueurs temporels par la valeur de l'écart-type des $\tau_{i,j}$).
Les marqueurs temporels de fin $t\_EAx\_end\_xxx$. sont déterminés de façon semblable.
Dans le cas particulier du mode "monitoring", on pourra en plus calculer des statistiques sur les marqueurs temporels, et d'autres caractéristiques extraites à différents instants successifs tout au long du signal.
On extrait également, pour chaque battement sélectionné et pour chaque composante EA1 et EA2 du signal EA, les valeurs d'amplitude pic à pic dans la fenêtre déterminée par les deux bornes, valeurs qui seront désignées PEA1 et PEA2. On peut ainsi calculer une valeur moyenne et un écart-type de PEA1 et PEA2 sur l'ensemble des valeurs obtenues sur les battements sélectionnés.
Dans le cas de la technique de segmentation basée sur un modèle auto-régressif récursif RAR (bloc 160" sur la Figure 2), il est possible d'extraire d'autres marqueurs temporels intéressants : on peut en effet détecter un certain nombre d'instants caractéristiques parmi lesquels un instant de rupture de fréquence ou un instant d'inflexion de fréquence, selon la technique utilisée (point de transition entre deux composantes fréquentielles d'EAx), et l'instant de fréquence maximum. On pourra aussi extraire des caractéristiques non temporelles, en estimant par exemple des modèles de variation de la fréquence au cours du temps (modèles hyperboliques, sigmoïdes, polynômes d'ordres supérieurs, exponentiels, linéaires par morceaux, etc.) et utiliser les paramètres de ces modèles (coefficients, constantes de temps, amplitude et phase des pôles, etc.) comme caractéristiques d'une composante EAx.
Une fois le signal EA moyen segmenté complètement (temps de début et de fin des composantes EA1 et EA2) on peut définir pour chaque composante un rapport signal sur bruit SNR :

$$SNR\_EAx = PEAx \, / \, (2 \times \sigma\_noise),$$

$\sigma\_noise$ étant l'écart-type du signal considéré comme "bruit", c'est-à-dire le signal contenu dans la fenêtre de signal utile EAx, à l'exception du segment correspondant à la composante EAx proprement dite. Cette situation est illustrée sur la Figure 11 pour la composante EA2, où l'on peut voir que le bruit à évaluer pour le calcul du rapport SNR est celui du signal contenu dans la fenêtre de signal utile EA2 à l'exception du segment correspondant à la composante EA2, allant de $t\_EA2\_start$ à $t\_EA2\_end$. Dans le cas où la segmentation de la composante EA2 aurait échoué, on considérera que le signal de type "bruit" est tout le signal contenu dans la fenêtre de signal utile EA2.

Les caractéristiques ainsi obtenues peuvent être combinées et utilisées (blocs 180 et 190 sur la Fig. 2) pour l'évaluation de la fonction cardiaque hémodynamique du patient.

En effet, le marqueur temporel de début associé à la composante EA1 est corrélé à l'instant d'ouverture de la valve aortique, tandis que le marqueur temporel de début associé à la composante EA2 est corrélé à l'instant de fermeture de la valve aortique. On peut donc facilement évaluer la période d'éjection, par calcul de l'intervalle séparant ces deux marqueurs.

On peut utiliser d'autres caractéristiques du signal ou une combinaison de caractéristiques pour évaluer d'autres indices de la fonction cardiaque.

Ces caractéristiques permettent de suivre l'évolution de paramètres hémodynamiques très importants, habituellement mesurés par échocardiographie ou lors d'examens invasifs (mesure de pression dans le ventricule gauche).

Ces indications peuvent en outre permettre d'évaluer la qualité d'une thérapie de resynchronisation délivrée au patient, la localisation d'un site de stimulation optimale, l'ajustement du délai interventriculaire, etc., comme cela a été exposé au début de la présente description.

Il est également possible d'établir des modèles linéaires ou non, appris sur une population incluant des patients insuffisants cardiaques et/ou des sujets sains. Ces modèles permettront, une fois déterminés les "timings" des composantes EAx (positions temporelles de leurs instants caractéristiques), d'estimer une valeur de "timings" valvulaires, tels que les instants d'ouverture/fermeture de la valve aortique, et les instants d'ouverture/fermeture de la valve mitrale.

## Revendications

1. Un dispositif d'analyse d'un signal EA d'accélération endocardiaque, ce dispositif comprenant :

   - des moyens de traitement, recevant en entrée un signal EA sur un cycle cardiaque, et délivrant en sortie au moins une donnée caractéristique corrélée à des paramètres temporels du cycle cardiaque et/ou aux performances hémodynamiques du myocarde,
   - des moyens de prétraitement, recevant en entrée un signal EA recueilli sur une séquence de cycles cardiaques et délivrant en sortie ledit signal EA sur un cycle appliqué en entrée auxdits moyens de traitement, dispositif **caractérisé en ce que** ces moyens de prétraitement comprennent :

     · des moyens (100) de découpage dudit signal EA recueilli sur la séquence de cycles cardiaques, par détermination de marqueurs de début de cycle isolant les cycles cardiaques successifs de ce signal, de manière à produire une série de sous-signaux EA chacun sur une durée d'un cycle cardiaque ;
     · des moyens séparateurs (110), configurés pour isoler dans chacun desdits sous-signaux EA au moins une composante EAx associée à l'un des bruits cardiaques (S1, S2, S3 ou S4), pour ce sous-signal ; et
     · des moyens corrélateurs (120, 120'), opérant sur ladite composante EAx, configurés pour rechercher un maximum de corrélation et opérer un recalage temporel relatif de chacun des sous-signaux de la série de sous-signaux EA par rapport à ce maximum, et configurés pour délivrer une composante EAx recalée associée à chaque sous-signal de ladite série de sous-signaux EA.

2. Le dispositif d'analyse d'un signal EA de la revendication 1, dans lequel :

   · les moyens séparateurs (110) sont des moyens configurés pour isoler dans chacun desdits sous-signaux EA : une composante EA1 associée au premier bruit cardiaque majeur (S1) pour ce sous-signal, et une composante EA2 associée au second bruit cardiaque majeur (S2) pour ce même sous-signal ; et
   · les moyens corrélateurs (120, 120') sont des moyens opérant de façon distincte sur chacune des composantes EA1 et EA2, configurés pour rechercher un maximum de corrélation et opérer un recalage temporel relatif de chacun des sous-signaux de la série de sous-signaux EA par rapport à ce maximum, et configurés pour délivrer une composante EA1 recalée et une composante EA2 recalée associée à chaque sous-signal de ladite série de sous-signaux EA.

3. Le dispositif d'analyse d'un signal EA de la revendication 2, dans lequel les moyens de prétraitement comprennent en outre :

· des moyens associateurs (130), configurés pour déterminer à partir des composantes EA1 et EA2 délivrées par les moyens corrélateurs une composante EA1 moyenne et une composante EA2 moyenne, et configurés pour combiner ces composantes EA1 et EA2 moyennes de manière à produire un signal EA global moyen sur un cycle.

4. Le dispositif d'analyse d'un signal EA de la revendication 1, dans lequel lesdits moyens de traitement comprennent des moyens configurés pour déterminer des marqueurs temporels d'instants caractéristiques du cycle cardiaque, à partir dudit signal EA global moyen sur un cycle délivré par les moyens de prétraitement.

5. Le dispositif d'analyse d'un signal EA de la revendication 4, dans lequel lesdits marqueurs temporels sont des marqueurs temporels corrélés aux instants d'ouverture et de fermeture des valves aortique, mitrale, pulmonaire et/ou tricuspide.

6. Le dispositif d'analyse d'un signal EA de la revendication 1, dans lequel lesdits moyens de découpage (100) comprennent en outre des moyens de calcul d'une durée médiane ou moyenne des cycles de ladite séquence de cycles cardiaques, et d'ajustement de la durée de chacun des sous-signaux EA à une même durée correspondant à ladite durée médiane ou moyenne ainsi calculée.

7. Le dispositif d'analyse d'un ignal EA de la revendication 2, comprenant en outre :

- des moyens (140) de calcul d'une valeur de contraste de l'amplitude crête-à-crête (PEA1 ou PEA2) de l'accélération endocardiaque dans la composante EA1 ou EA2 respective, ces moyens opérant de façon distincte sur chacune des composantes EA1 et EA2.

8. Le dispositif d'analyse d'un signal EA de la revendication 2, comprenant en outre :

- des moyens (140) de calcul d'une valeur d'entropie de la composante EA1 ou EA2, ces moyens opérant de façon distincte sur chacune des composantes EA1 et EA2.

9. Le dispositif d'analyse d'un signal EA de la revendication 3, dans lequel lesdits moyens de traitement comprennent des moyens (160, 160') de calcul d'une enveloppe énergétique de Shannon, ou d'une enveloppe par filtrage homomorphique, appliqué audit signal EA global moyen sur un cycle délivré par les moyens de prétraitement.

10. Le dispositif d'analyse d'un signal EA de la revendication 1, dans lequel lesdits moyens de traitement comprennent des moyens (160") d'estimation d'une fréquence fondamentale instantanée dudit signal EA global moyen sur un cycle délivré par les moyens de prétraitement, par application d'un modèle auto-régressif estimé de manière récursive.

11. Le dispositif d'analyse d'un signal EA de la revendication 10, dans lequel lesdits moyens de traitement comprennent en outre des moyens aptes à détecter des données caractéristiques parmi : instant de rupture de fréquence ou instant d'inflexion de fréquence ; instant de fréquence maximum ; données non temporelles caractérisées à partir de modèles estimés de variation de la fréquence au cours du temps.

12. Le dispositif d'analyse d'un signal EA de la revendication 3, comprenant en outre des moyens (150) configurés pour commander la sélection automatique d'un parmi plusieurs moyens de traitement différents, ou d'une combinaison desdits traitements, en fonction de critères appliqués à ladite composante EA1 moyenne et/ou à ladite composante EA2 moyenne délivrée par les moyens associateurs.

13. Le dispositif d'analyse d'un signal EA de la revendication 12, comprenant en outre des moyens configurés pour combiner entre eux, par une combinaison linéaire ou non-linéaire, une pluralité de résultats produits par une pluralité correspondante de traitement différents concurremment mis en oeuvre, pour au moins l'une desdites données caractéristiques corrélées à des paramètres temporels du cycle cardiaque et/ou aux performances hémodynamiques du myocarde.

**14.** Le dispositif d'analyse d'un signal EA de la revendication 3, dans lequel lesdits moyens de traitement comprennent des moyens (170) configurés pour déterminer, à partir dudit signal EA global moyen sur un cycle délivré par les moyens de prétraitement, au moins un paramètre parmi : variabilité cycle-à-cycle de marqueurs temporels d'un instant caractéristique du cycle cardiaque, fréquence médiane du signal dans des intervalles prédéterminés, énergie cumulée du signal dans des intervalles prédéterminés, énergie maximale du signal dans des intervalles prédéterminés.

**15.** Le dispositif d'analyse d'un signal EA de la revendication 2, comprenant en outre :

- des moyens de calcul d'une valeur de rapport signal/bruit des composantes EA1 ou EA2 respectives, ces moyens opérant de façon distincte sur chacune des composantes EA1 et EA2.

**16.** Le dispositif d'analyse d'un signal EA de la revendication 1, dans lequel lesdits moyens de découpage (100) comprennent en outre des moyens de détection de la présence d'extrasystoles sur ladite séquence de cycles cardiaques, et d'élimination des sous-signaux EA relatifs à des cycles affectés par les extrasystoles ainsi détectées.

**17.** Le dispositif d'analyse d'un signal EA de la revendication 1, comprenant un mode "monitoring" de suivi de l'évolution temporelle des caractéristiques du signal EA sur une fenêtre d'analyse glissante s'étendant sur au moins un battement cardiaque.

**18.** Le dispositif d'analyse d'un signal EA de la revendication 16, comprenant en outre des moyens de calcul de statistiques sur les marqueurs temporels, et de calcul de caractéristiques extraites à différents instants successifs tout au long du signal.

**Patentansprüche**

**1.** Vorrichtung zur Analyse eines endokardialen Beschleunigungssignals EA, wobei diese Vorrichtung aufweist:

- Verarbeitungsmittel, die am Eingang ein Signal EA über einen Herzzyklus hinweg empfangen und am Ausgang wenigstens ein charakteristisches Datenelement liefern, das mit zeitlichen Parametern des Herzzyklus und/oder mit den hämodynamischen Eigenschaften des Myokards korreliert ist,
- Vorverarbeitungsmittel, die am Eingang ein Signal EA empfangen, das über eine Reihe von Herzzyklen hinweg aufgenommen wird, und am Ausgang das Signal EA über einen Zyklus liefern, das als Eingang an die Verarbeitungsmittel angelegt wird,

wobei die Vorrichtung **dadurch gekennzeichnet ist, dass** diese Vorverarbeitungsmittel aufweisen:

• Mittel (100) zur Segmentierung des Signals EA, das über die Reihe von Herzzyklen hinweg aufgenommen wird, durch Bestimmung von Marken des Zyklusanfangs, welche die aufeinander folgenden Herzzyklen dieses Signals isolieren, derart, dass eine Reihe von Untersignalen EA erzeugt wird, die sich jeweils über eine Dauer eines Herzzyklus erstrecken;
• Trennmittel (110), die dafür ausgelegt sind, in jedem der Untersignale EA wenigstens eine Komponente EAx zu isolieren, die einem der Herztöne (S1, S2, S3 oder S4) für dieses Untersignal zugeordnet ist; und
• Korreliermittel (120, 120'), die auf der Komponente EAx arbeiten, die dafür ausgelegt sind, ein Korrelationsmaximum zu suchen und eine relative zeitliche Ausrichtung jedes der Untersignale der Reihe von Untersignalen EA in Bezug auf dieses Maximum zu bewirken, und die dafür ausgelegt sind, für jedes Untersignal der Reihe von Untersignalen EA eine zugeordnete ausgerichtete Komponente EAx zu liefern.

**2.** Vorrichtung zur Analyse eines Signals EA nach Anspruch 1, wobei:

• die Trennmittel (110) Mittel sind, die dafür ausgelegt sind, in jedem der Untersignale EA zu isolieren: eine Komponente EA1, die dem ersten Hauptherzton (S1) für dieses Untersignal zugeordnet ist, und eine Komponente EA2, die dem zweiten Hauptherzton (S2) für dasselbe Untersignal zugeordnet ist; und

• die Korreliermittel (120, 120') Mittel sind, die auf unterschiedliche Weise auf jeder der Komponenten EA1 und EA2 arbeiten, die dafür ausgelegt sind, ein Korrelationsmaximum zu suchen und eine relative zeitliche Ausrichtung jedes der Untersignale der Reihe von Untersignalen EA in Bezug auf dieses Maximum zu bewirken, und die dafür ausgelegt sind, für jedes Untersignal der Reihe von Untersignalen EA eine zugeordnete ausgerichtete Komponente EA1 und eine zugeordnete ausgerichtete Komponente EA2 zu liefern.

3. Vorrichtung zur Analyse eines Signals EA nach Anspruch 2, wobei die Vorverarbeitungsmittel außerdem aufweisen:

• Vereinigungsmittel (130), die dafür ausgelegt sind, ausgehend von den Komponenten EA1 und EA2, die von den Korreliermitteln geliefert wurden, eine mittlere Komponente EA1 und eine mittlere Komponente EA2 zu bestimmen, und dafür ausgelegt sind, diese mittleren Komponenten EA1 und EA2 zu kombinieren, um ein mittleres globales Signal über einem Zyklus zu erzeugen.

4. Vorrichtung zur Analyse eines Signals EA nach Anspruch 1, wobei die Verarbeitungsmittel Mittel aufweisen, die dafür ausgelegt sind, ausgehend von dem mittleren globalen Signal EA über einem Zyklus, das von den Vorverarbeitungsmitteln geliefert wird, Zeitmarken von charakteristischen Zeitpunkten des Herzzyklus zu bestimmen.

5. Vorrichtung zur Analyse eines Signals EA nach Anspruch 4, wobei die Zeitmarken Zeitmarken sind, die mit den Zeitpunkten des Öffnens und des Schließens der Aortenklappe, Mitralklappe, Pulmonalklappe und/oder Trikuspidalklappe korreliert sind.

6. Vorrichtung zur Analyse eines Signals EA nach Anspruch 1, wobei die Mittel zur Segmentierung (100) außerdem Mittel zur Berechnung eines Medians der Dauer oder einer mittleren Dauer der Zyklen der Reihe von Herzzyklen und zur Einstellung der Dauer jedes der Untersignale EA auf ein und dieselbe Dauer, die diesem so berechneten Median der Dauer oder dieser so berechneten mittleren Dauer entspricht, aufweisen.

7. Vorrichtung zur Analyse eines Signals EA nach Anspruch 2, welche außerdem aufweist:

- Mittel (140) zur Berechnung eines Kontrastwertes der Peak-zu-Peak-Amplitude (PEA1 oder PEA2) der endokardialen Beschleunigung in der jeweiligen Komponente EA1 oder EA2, wobei diese Mittel auf unterschiedliche Weise auf jeder der Komponenten EA1 und EA2 arbeiten.

8. Vorrichtung zur Analyse eines Signals EA nach Anspruch 2, welche außerdem aufweist:

- Mittel (140) zur Berechnung eines Entropiewertes der Komponente EA1 oder EA2, wobei diese Mittel auf unterschiedliche Weise auf jeder der Komponenten EA1 und EA2 arbeiten.

9. Vorrichtung zur Analyse eines Signals EA nach Anspruch 3, wobei die Verarbeitungsmittel Mittel (160, 160') zur Berechnung einer Shannon-Energiehüllkurve oder einer Hüllkurve durch homomorphe Filterung, angewendet auf das mittlere globale Signal EA über einem Zyklus, das von den Vorverarbeitungsmitteln geliefert wird, aufweisen.

10. Vorrichtung zur Analyse eines Signals EA nach Anspruch 1, wobei die Verarbeitungsmittel Mittel (160") zur Schätzung einer momentanen Grundfrequenz des mittleren globalen Signals EA über einem Zyklus, das von den Vorverarbeitungsmitteln geliefert wird, durch Anwendung eines rekursiv geschätzten autoregressiven Modells aufweisen.

11. Vorrichtung zur Analyse eines Signals EA nach Anspruch 10, wobei die Verarbeitungsmittel außerdem Mittel aufweisen, die geeignet sind, charakteristische Datenelemente zu erfassen, die zu folgender Gruppe gehören: Zeitpunkt der Frequenzunterbrechung oder Zeitpunkt des Frequenzknicks; Zeitpunkt maximaler Frequenz; nicht zeitbezogene Daten, die ausgehend von Schätzmodellen der Frequenzänderung im Laufe der Zeit charakterisiert sind.

12. Vorrichtung zur Analyse eines Signals EA nach Anspruch 3, welche außerdem Mittel (150) aufweist, die dafür ausgelegt sind, die automatische Auswahl eines von mehreren verschiedenen Verarbeitungsmitteln oder einer Kombination dieser Verarbeitungen in Abhängigkeit von Kriterien zu steuern, die auf die mittlere Komponente EA1 und/oder auf die mittlere Komponente EA2 angewendet werden, die von den Vereinigungsmitteln geliefert wird.

13. Vorrichtung zur Analyse eines Signals EA nach Anspruch 12, welche außerdem Mittel aufweist, die dafür ausgelegt sind, mehrere Ergebnisse, die durch eine entsprechende Vielzahl gleichzeitig durchgeführter verschiedener Verarbeitungen erzeugt wurden, durch eine Linearkombination oder eine nichtlineare Kombination miteinander zu kombinieren, für wenigstens eines der charakteristischen Datenelemente, die mit zeitlichen Parametern des Herzzyklus und/oder mit den hämodynamischen Eigenschaften des Myokards korreliert sind.

14. Vorrichtung zur Analyse eines Signals EA nach Anspruch 3, wobei die Verarbeitungsmittel Mittel (170) aufweisen, die dafür ausgelegt sind, ausgehend von dem mittleren globalen Signal EA über einem Zyklus, das von den Vorverarbeitungsmitteln geliefert wird, mindestens einen Parameter von folgenden zu bestimmen: Veränderlichkeit von Zyklus zu Zyklus von Zeitmarken eines charakteristischen Zeitpunkts des Herzzyklus, Medianwert der Frequenz des Signals in vorbestimmten Intervallen, kumulierte Energie des Signals in vorbestimmten Intervallen, maximale Energie des Signals in vorbestimmten Intervallen.

15. Vorrichtung zur Analyse eines Signals EA nach Anspruch 2, welche außerdem aufweist:

   - Mittel zur Berechnung eines Wertes des Signal-Rausch-Verhältnisses der jeweiligen Komponenten EA1 oder EA2, wobei diese Mittel auf unterschiedliche Weise auf jeder der Komponenten EA1 und EA2 arbeiten.

16. Vorrichtung zur Analyse eines Signals EA nach Anspruch 1, wobei die Mittel zur Segmentierung (100) außerdem Mittel zur Erkennung des Vorhandenseins von Extrasystolen auf der Reihe von Herzzyklen und zur Eliminierung der Untersignale EA, welche Zyklen betreffen, die durch die so erkannten Extrasystolen beeinflusst sind, aufweisen.

17. Vorrichtung zur Analyse eines Signals EA nach Anspruch 1, welche einen "Überwachungs"-Modus zur Verfolgung der zeitlichen Veränderung der Kenngrößen des Signals EA in einem gleitenden Analysefenster, das sich über mindestens einen Herzschlag erstreckt, aufweist.

18. Vorrichtung zur Analyse eines Signals EA nach Anspruch 16, welche außerdem Mittel zur Berechnung von Statistiken über die Zeitmarken und zur Berechnung von Kenngrößen, die zu verschiedenen aufeinander folgenden Zeitpunkten über die gesamte Dauer des Signals extrahiert wurden, aufweist.

**Claims**

1. A device for analysing an endocardial acceleration EA signal, said device comprising:

   - processing means, receiving as an input an EA signal over one cardiac cycle, and delivering as an output at least one characteristic data correlated to time parameters of the cardiac cycle and/or to the hemodynamic performances of the myocardium,
   - pre-processing means, receiving as an input an EA signal collected over a sequence of cardiac cycles and delivering as an output said EA signal overs one cycle applied at the input of said processing means,

   the device being **characterized in that** said pre-processing means comprise:

   . means (100) for cutting said EA signal collected over the sequence of cardiac cycles, by determining markers of beginning of cycle isolating the successive cardiac cycles of this signal, so as to produce a series of EA sub-signals, each over a duration of one cardiac cycle;
   . separating means (110), configured to isolate in each of said EA sub-signals at least one component EAx related to one of the cardiac noises (S1, S2, S3 or S4) for this sub-signal; and
   . correlating means (120, 120'), operating on said EAx component, configured to search for a maximum of correlation and to operate a relative retiming of each of the sub-signals of the series of EA sub-signals with respect to this maximum, and configured to deliver a retimed EAx component related to each sub-signal of said series of EA sub-signals.

2. The device for analysing an EA signal of claim 1, wherein:

   - the separator means (110) are means configured to isolate in each of said EA sub-signals: a component EA1 related to the first major cardiac noise (S1) for this sub-signal, and a component EA2 related to the second major cardiac noise (S2) for this same sub-signal; and
   - the correlating means (120, 120') are means operating distinctively on each of the components EA1 and EA2, configured to search for a maximum of correlation and to operate a relative retiming of each of the sub-signals of the series of EA sub-signals with respect to this maximum, and configured to deliver a retimed EA1 component and a retimed EA2 component related to each sub-signal of said series of EA sub-signals.

3. The device for analysing an EA signal of claim 2, wherein the pre-processing means further comprise:

   - associating means (130), configured to determine based on the components EA1 and EA2 delivered by the correlating means a mean EA1 component and a mean EA2 component, and configured to combine these mean EA1 and EA2 components to produce a mean global EA signal over one cycle.

4. The device for analysing an EA signal of claim 1, wherein said processing means comprise means configured to determine time markers of characteristic instants of the cardiac cycle, based on said mean global EA signal over one cycle delivered by the pre-processing means.

5. The device for analysing an EA signal of claim 4, wherein said time markers are time markers correlated to the instants of opening and closure of the aortic, mitral, pulmonary and/or tricuspid valves.

6. The device for analysing an EA signal of claim 1, wherein said cutting means (100) further comprise means for calculating a median or mean duration of the cycles of said sequence of cardiac cycles, and for adjusting the duration of each of the EA sub-signals to a same duration corresponding to said thus-calculated median or mean duration.

7. The device for analysing an EA signal of claim 2, further comprising:

   - means (140) for calculating a contrast value of the peak-to-peak amplitude (PEA1 or PEA2) of the endocardial acceleration in the respective component EA1 or EA2, these means operating distinctively on each of these components EA1 and EA2.

8. The device for analysing an EA signal of claim 2, further comprising:

   - means (140) for calculating a value of entropy of the component EA1 or EA2, these means operating distinctively on each of these components EA1 and EA2.

9. The device for analysing an EA signal of claim 3, wherein said processing means comprise means (160, 160') for calculating a Shannon energetic envelope, or an homomorphic filtering envelope, applied to said mean global EA signal over a cycle delivered by the pre-processing means.

10. The device for analysing an EA signal of claim 1, wherein said processing means comprise means (160") for estimating an instantaneous fundamental frequency of said mean global EA signal over a cycle delivered by the pre-processing means, by applying a recursively estimated auto-regressive model.

11. The device for analysing an EA signal of claim 10, wherein said processing means further comprise means adapted to detect characteristic data among: instant of frequency break or instant of frequency inflection; instant of maximum frequency; non-time data characterized based on estimated models of variation of the frequency over time.

12. The device for analysing an EA signal of claim 3, further comprising means (150) configured to control the automatic selection of one of several different processing means, or a combination of said processing means, as a function of criteria applied to said mean EA1 component and/or to said mean EA2 component delivered by the associating means.

13. The device for analysing an EA signal of claim 12, further comprising means configured to combined between each other, by a linear or non-linear combination, a plurality of results produced by a corresponding plurality of different processing means implemented concurrently, for at least one of said characteristic data correlated to time parameters of the cardiac cycle and/or to the hemodynamic performances of the myocardium.

14. The device for analysing an EA signal of claim 3, wherein said processing means comprise means (170) configured to determine, based on the mean global EA signal over one cycle delivered by the pre-processing means, at least one parameter among: cycle-by-cycle variability of the time markers of a characteristic instant of the cardiac cycle, median frequency of the signal in predetermined intervals, cumulated energy of the signal in predetermined intervals, maximum energy of the signal in predetermined intervals.

**15.** The device for analysing an EA signal of claim 2, further comprising:

    - means for calculating a signal/noise ratio value of the respective components EA1 and EA2, these means operating distinctively on each of the components EA1 and EA2.

**16.** The device for analysing an EA signal of claim 1, wherein said cutting means (100) further comprise means for detecting the presence of extrasystoles in said sequence of cardiac cycles, and for eliminating the EA sub-signal relating to cycles affected by the thus-detected extrasystoles.

**17.** The device for analysing an EA signal of claim 1, comprising a "monitoring" mode for following the evolution over time of the characteristics of the EA signal in a sliding analysis window extending over at least one cardiac beat.

**18.** The device for analysing an EA signal of claim 16, further comprising means for calculating statistics on the time markers, and for calculating characteristics extracted at different successive instants all over the signal.

FIG_1

FIG_3

## FIG_2

```
┌─────────────────────────────────────────────┐
│  DECOUPAGE SIGNAL EN CYCLES CARDIAQUES       │──100
└─────────────────────────────────────────────┘
                      │
┌─────────────────────────────────────────────┐
│  SELECTION DES COMPOSANTES EA1 ET EA2        │──110
│  SATISFAISANT LES CONDITIONS DE CORRELATION  │
└─────────────────────────────────────────────┘
         │ 120                    │ 120'
┌──────────────────────────┐  ┌──────────────────────────────┐
│ RECALAGE TEMPOREL DES     │  │ RECALAGE TEMPOROREL DES       │
│ NCYCLES EAX               │  │ NCYCLES EAX                   │
└──────────────────────────┘  └──────────────────────────────┘
         │                                  │
┌─────────────────────────────────────────────┐
│  GENERATION DU CYCLE MOYEN                   │──130
└─────────────────────────────────────────────┘
                      │
┌─────────────────────────────────────────────┐
│  CALCUL DES VALEURS DE CONTRASTE, PEAX       │──140
│  ET EAX_CORRELATION                          │
└─────────────────────────────────────────────┘
                      │
┌─────────────────────────────────────────────┐
│  DECISION DE(S) ALGO(S) A APPLIQUER          │──150
└─────────────────────────────────────────────┘
        ╱              │               ╲
   160 ╱          160' │            160" ╲
┌──────────┐  ┌─────────────────┐  ┌────────┐
│ SHANNON  │  │ HOMOMORPHIQUE   │  │ RAR    │
└──────────┘  └─────────────────┘  └────────┘
     │              │                  │
┌─────────────────────────────────────────────┐
│  EXTRACTION D'AUTRES CARACTERISTIQUES        │──170
└─────────────────────────────────────────────┘
                      │
┌─────────────────────────────────────────────┐
│  COMBINAISON DES DIFFERENTES CARACTÉRISTIQUES│──180
└─────────────────────────────────────────────┘
                      │
┌─────────────────────────────────────────────┐
│  EVALUATION DE LA FONCTION CARDIAQUE         │──190
│  HEMODYNAMIQUE GLOBALE                       │
└─────────────────────────────────────────────┘
```

## FIG_4

EA1

EA2

## FIG_5

(a)

0,01
0,08
0,06
0,04
0,02
0
-0,02
-0,04
-0,06
-0,08
-0,01

450    500    550    600    650    700    750    800

t (échantillons)

Coefficient
de
corrélation

(b)

0,5
0,4
0,2
0
-0,2
-0,4
-0,6

-200    -150    -100    -50    0    50    100    150    200

t(échantillons)

FIG_6

FIG_7

FIG_8

FIG_9a

FIG_9b

FIG_10

FIG_11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1108446 A1
- EP 0108446 A1
- EP 0515319 A1
- EP 0655260 A1
- EP 1736203 A1
- US 7139609 B1
- EP 1741387 A1